# FASCICULE DE BREVET EUROPEEN

(11) **EP 0 651 815 B1**
(45) Date de publication et mention de la délivrance du brevet: **09.04.1997**
(21) Numéro de dépôt: 91913605.1
(22) Date de dépôt: 17.07.1991
(51) Int. Cl.: C12N 15/86, C12N 15/33, C12N 15/52, C12N 7/01

(54) **BACULOVIRUS MODIFIE, SON PROCEDE D'OBTENTION, ET VECTEURS D'EXPRESSION OBTENUS A PARTIR DUDIT BACULOVIRUS**
MODIFIZIERTES BACULOVIRUS, VERFAHREN ZU SEINER HERSTELLUNG, UND DESSEN EXPRESSIONSVEKTOREN
MODIFIED BACULOVIRUS, METHOD FOR OBTAINING IT, AND EXPRESSION VECTORS OBTAINED FROM SAID BACULOVIRUS

(30) Priorité: 18.07.1990 FR 9009143
(43) Date de publication de la demande: 10.05.1995
(73) Titulaire: INSTITUT NATIONAL DE LA RECHERCHE AGRONOMIQUE, F-75341 Paris Cédex 07 (FR); CENTRE NATIONAL DE LA RECHERCHE SCIENTIFIQUE (CNRS), 75794 Paris Cédex 16 (FR)
(72) Inventeur: DEVAUCHELLE, Gérard, F-30380 Saint-Christol-lès-Alès (FR); CERUTTI, Martine, F-30380 Saint-Christol-lès-Alès (FR); CAHOREAU, Claire, F-30100 Nîmes (FR); MARTIN, Marianne 15 bis, chemin de l'Escalette, F-30700 Uzès (FR)
(74) Mandataire: Ores, Irène
(86) Numéro de dépôt international: FR9100588
(87) Numéro de publication internationale: WO9201801

(56) Documents cités:
- EP-A- 0 127 839
- Gene, volume 70, no. 1, 15 octobre 1988, Elsevier Science Publishers B.V. (Amsterdam, NL) C. Rankin et al.: "Eight base pairs encompassing the transcriptional start point are the major determinant for baculovirus polyhedrin gene expression", pages 39-49, voir le document en entier
- Journal of General Virology, volume 71, no. 7, juillet 1990, (Colchester, GB) U. Weyer et al.: "Analysis of very late gene expression by Autographa californica nuclear polyhedrosis virus and the further development of multiple expression vectors", pages 1525-1534, voir l'article en entier
- Journal of General Virology, volume 69, no. 4, avril 1988 (Colchester, GB) J.M. Vlak et al.: "Functional studies on the p10 gene of Autographa californica nuclear polyhedrosis virus using a recombinant expressing a p10-beta- galactosidase fusion gene", pages 765-776, voir l'article en entier
- Gene, volume 75, no. 1, 30 janvier 1989, Elsevier Science Publishers B.V. (Amsterdam, NL) K. Iatrou et al.: "Bombyx mori nuclear polyhedrosis virus-based vectors for expressing passenger genes in silkmoth cells under viral or cellular promoter control", pages 59-71, voir l'article en entier (cité dans la demande)
- Biological Abstracts Database, abrégé no. 88037377, J. Qin et al.: "Studies on the control region of the P10 gene of the Autographa-californica nuclear polyhedrosis virus", & J. Gen. Virology, 1989, vol. 70, no. 5, p. 1273-1280, voir abregé
- Biotechnology Abstracts Database, abrégé no. 88-01644, R.D. Possee et al.: "Analysis of the polyhedrin gene promoter of the Autographa californica nuclear polyhedrosis virus. Potential baculo virus vector for foreign gene expression", & Nucleic Acids Res. 1987, vol. 15, no. 24, p. 10233-48, voir l'abrégé

## Description

La présente Invention est relative à des vecteurs d'expression obtenus à partir de baculovirus modifiés, dans lesquels l'un des deux promoteurs tardifs forts du baculovirus sauvage est inactif.

Les baculovirus sont actuellement utilisés dans de très nombreux laboratoires comme vecteurs d'expression de gènes. En effet, ces virus possèdent les avantages suivants : ils permettent l'insertion de longs segments d'ADN et possèdent en outre deux promoteurs tardifs forts, le promoteur de la polyédrine, et le promoteur de la protéine P10, qui sont capables d'induire un niveau d'expression extrêmement élevé des gènes placés sous leur contrôle.

La publication de L.K. MILLER [CHAPITRE 14 "A VIRUS VECTOR FOR GENETIC ENGINEERING IN INVERTEBRATES" du Manuel "GENETIC ENGINEERING IN THE PLANTS SCIENCES" (1981) N.J. PANAPOULOS, ed., Praeger Pub. New York, p. 203-224] décrit les avantages de l'utilisation des baculovirus, et en particulier du baculovirus *Autographa californica* (AcNPV), comme vecteurs d'expression de gènes étrangers dans des cellules-hôtes d'insectes. Le baculovirus AcNPV décrit dans cette publication possède deux formes matures respectivement appelées forme non occlusive (NOV) et forme occlusive (OV). Les formes non occlusives sont responsables de la transmission du virus en culture cellulaire ou à l'intérieur d'un même organisme. Les formes occlusives sont responsables de la transmission du virus d'un organisme à un autre. Ces formes occlusives sont constituées de virions enveloppés dans une matrice protéique cristalline essentiellement constituée d'une seule protéine : la polyédrine.

Il a été démontré que le gène de la polyédrine possédait un promoteur exceptionnellement fort qui permettait d'assurer un niveau d'expression élevé du gène placé sous son contrôle. MILLER signale donc qu'il serait particulièrement avantageux d'utiliser les baculovirus comme vecteurs d'expression, en insérant sous contrôle du promoteur de la polyédrine l'ADN exogène que l'on désire exprimer.

D'autres travaux [SMITH et SUMMERS., J.Virol. 45, 215-225 (1983)] ont montré que le virus AcNPV possédait un autre promoteur tardif fort, qui est le promoteur du polypeptide 10kDa, ou protéine P10.

En outre la capside des baculovirus peut contenir de grandes quantités d'ADN et ne constitue donc pas une limite au nombre ou à la longueur des gènes insérés. En conséquence, de très nombreux vecteurs d'expression dérivés des baculovirus ont été proposés. Par exemple, la Demande de Brevet Européen 127 839, (Invention SUMMERS), décrit un procédé permettant la production d'un vecteur d'expression recombinant, issu de baculovirus, en passant par l'intermédiaire d'un vecteur de transfert renfermant un fragment d'ADN viral qui contient le promoteur du gène de la polyédrine, en aval duquel est inséré le gène étranger. Ce vecteur de transfert est ensuite recombiné avec l'ADN du baculovirus sauvage et les recombinants possédant le gène étranger sous contrôle du promoteur de la polyédrine sont sélectionnés.

La Demande de Brevet Européen 340 359 (Inventeurs PAGE et RODGERS) décrit des vecteurs de transfert dérivés des baculovirus, dans lesquels le codon d'initiation ATG de la polyédrine est supprimé, de façon à ce que la séquence codant pour la polyédrine ne puisse pas être traduite, et qui portent un site de restriction convenant à l'insertion d'un gène exogène en aval de l'extrémité N-terminale du gène de la polyédrine, et à proximité immédiate de ladite extrémité.

L'équipe des Inventeurs a précédemment mis au point un procédé de production d'un baculovirus modifié, utilisable comme vecteur d'expression, dans lequel on insère directement et sans recourir à un vecteur de transfert, un site de restriction approprié, en aval d'un promoteur tardif fort. Le vecteur ainsi obtenu peut être chargé, à l'emplacement du site de restriction introduit, avec le gène que l'on désire faire exprimer ; ce procédé fait l'objet de la Demande de Brevet Européen 345 152.

Dans tous les procédés connus, le gène à exprimer est inséré sous le contrôle de l'un des deux promoteurs tardifs forts présents dans le génome du baculovirus sauvage, tandis que l'autre promoteur continue à fonctionner normalement.

Certaines constructions dérivées de baculovirus par inactivation d'un promoteur tardif fort sont connues dans l'art antérieur ; RANKIN et al. [GENE, 70, 39-49 (1988)] décrivent différentes mutations du promoteur de la polyédrine ayant pour effet d'inhiber à des degrés variés, l'expression d'un gène placé sous contrôle dudit promoteur. IATROU et al. [GENE, 75, 59-71 (1989)] décrivent l'obtention de baculovirus dans lequel le promoteur de la polyédrine a été inactivé par délétion. Ils proposent l'utilisation de ces baculovirus comme vecteurs de transfert, pour l'obtention de constructions dans lesquelles on souhaite placer un gène exogène sous le contrôle de son propre promoteur, et non sous celui du promoteur de la polyédrine.

Des constructions dans lesquelles le promoteur de la protéine P10 est inactif ont également été réalisées ; QUIN et al. [J. Gen. Virol. 70, 1273-1280 (1989)] décrivent ainsi des plasmides recombinants comprenant des constructions dans lesquelles un gène exogène est placé sous le contrôle de différents mutants du promoteur de la P10. Certaines de ces mutations ont pour effet l'inactivation totale du promoteur.

Toutefois, ces constructions n'ont été réalisées que sur des plasmides recombinants. En effet, le promoteur du gène de la P10 recouvre une partie de la séquence codant pour la protéine P26, et il est généralement considéré qu'une mutation dans ce promoteur ne permet pas d'obtenir de baculovirus viables.

D'autre part, il a été proposé, pour augmenter l'expression de gènes dans des vecteurs d'expression dérivés de baculovirus, de multiplier, dans un même vecteur, le nombre de copies du gène et le nombre de promoteurs. Par exemple, la Demande de Brevet Européen 0 127 839 suggère d'insérer dans un même baculovirus des copies d'un gène sous contrôle du promoteur de la polyédrine, d'autres copies sous contrôle du promoteur de la P10, et éventuellement, encore d'autres copies à d'autres emplacements du génome, chaque copie dudit gène étant sous contrôle d'un promoteur de baculovirus, ou bien de son propre promoteur.

Or, les Inventeurs ont découvert, de manière inattendue, que la suppression ou l'inactivation de l'un des deux promoteurs tardifs forts du baculovirus sauvage avait pour conséquence d'augmenter l'expression du gène placé sous le contrôle du promoteur restant.

La présente invention a pour but de pourvoir à de nouveaux vecteurs d'expression, construits à partir de baculovirus modifiés, et dans lesquels un seul des deux promoteurs tardifs présents dans le baculovirus sauvage est actif.

La présente invention a pour objet des vecteurs d'expression, caractérisés en ce qu'il sont obtenus à partir d'un baculovirus modifié dans lequel un des deux promoteurs tardifs forts présents dans le génome du baculovirus sauvage est inactif, et en ce que la séquence placée sous le contrôle du promoteur tardif fort actif dans ledit baculovirus modifié est différente de la séquence correspondante du baculovirus sauvage.

Au sens de la présente Invention, "séquence différente de la séquence correspondante du baculovirus sauvage" signifie en particulier que la séquence qui, chez le baculovirus sauvage, est contrôlée par le promoteur considéré, a fait l'objet de modifications visant à permettre l'expression d'un gène exogène sous contrôle dudit promoteur. De telles modifications comprennent par exemple l'insertion d'une séquence exogène (gène que l'on désire faire exprimer, séquence portant un ou plusieurs sites de restriction, etc ...) dans ladite séquence du baculovirus sauvage, ou à la place de tout ou partie de celle-ci.

Selon un mode de réalisation préféré d'un vecteur d'expression conforme à l'Invention, le promoteur inactif est celui du gène de la polyédrine et le promoteur actif est celui du gène de la protéine P10.

Selon un autre mode de réalisation préféré d'un vecteur d'expression conforme à l'Invention, le promoteur inactif est celui du gène de la protéine P10, et le promoteur actif est celui du gène de la polyédrine.

L'Invention a également pour objet des baculovirus modifiés utilisables pour l'obtention de vecteurs d'expression tels que définis plus haut.

Dans ce cadre, l'Invention englobe les baculovirus modifiés, infectieux, et dans lesquels le promoteur du gène de la protéine P10 est inactif.

Chez les baculovirus AcNPV (Autographa Californica Nuclear Polyhedrosis Virus), et GmNPV (Galleria mellonella Nuclear Polyhedrosis Virus), le gène du polypeptide P10, qui est situé dans le fragment de restriction EcoRI P, est encadré en 5', par un gène codant pour un polypeptide dénommé P26, et dans la région 3', par un gène codant pour un polypeptide dénommé P74. Le promoteur du gène de P10 se trouve à l'extrémité 3' du gène de P26, dans la région codante de ce dernier.

Les Inventeurs ont procédé à la délétion de la région comprise entre un site XhoI (position +569 du gène P26) et un site BglII (position +152 du gène P10), puis ont religué ces sites, après réparation par l'enzyme de Klenow. La région correspondant au promoteur et à l'extrémité 5' de P10 est ainsi délétée. Un gène chimérique constitué des 569 premières bases de P26 et des 130 dernières bases de P10 est obtenu ; ce gène est fonctionnel, et les baculovirus modifiés de la sorte sont parfaitement viables et produisent plus de polyédrine que le virus sauvage.

Selon un mode de réalisation préféré de la présente Invention, le génome dudit baculovirus modifié est dépourvu d'une séquence d'ADN comprise entre le site XhoI situé à +569pb du codon d'initiation ATG de la séquence codant pour le polypeptide P26 et le site BglII, situé à +152pb du codon d'initiation ATG de la séquence codant pour le polypeptide P10.

L'Invention englobe également les baculovirus suivants, dans lesquels le promoteur du gène de la polyédrine est inactif :
- un baculovirus modifié dont le génome est dépourvu d'une séquence d'ADN comprise entre le site EcoRV situé à -95 pb et le site SspI situé à +910 pb du codon d'initiation ATG de la séquence de la polyédrine.
- un baculovirus modifié dont le génome est dépourvu d'une séquence d'ADN comprise entre le site EcoRV situé à -95 pb et le site KpnI situé à +633 pb du codon d'initiation ATG de la séquence de la polyédrine.
- un baculovirus modifié dont le génome est dépourvu d'une séquence d'ADN comprise entre le site EcoRV situé à -95 pb et le site Eco47III situé à +733 pb du codon d'initiation ATG de la séquence codant pour la polyédrine.

Les Inventeurs ont constaté que les baculovirus ainsi modifiés se multiplient bien en culture cellulaire, et ne produisent plus la protéine dont le promoteur est inactivé, mais en revanche, synthétisent en plus grande quantité que le baculovirus sauvage, celle dont le promoteur est actif.

Selon un autre mode de réalisation préféré de la présente invention, les baculovirus modifiés contiennent une séquence marqueur, placée sous le contrôle du promoteur actif.

Au sens de la présente Invention, on entend par "séquence marqueur" une séquence dont l'expression confère au baculovirus modifié un phénotype aisément identifiable. L'insertion ultérieure d'une séquence exogène à l'intérieur de ladite séquence entraîne l'annulation dudit phénotype, ce qui permet la sélection des baculovirus ayant intégré ladite séquence exogène.

Selon une disposition particulièrement avantageuse de ce mode de réalisation, la séquence marqueur est constituée par la séquence codant pour la polyédrine, laquelle séquence est placée sous le contrôle du promoteur du gène de la protéine P10. Les baculovirus modifiés obtenus de la sorte recouvrent leur capacité à produire des inclusions. Une souche de baculovirus obtenue conformément à cette disposition a été déposée, en date du 17 Juillet 1990, auprès de la Collection Nationale de Microorganismes, tenue par l'Institut Pasteur à Paris. Cette souche porte le numéro de dépôt I-978.

Selon une autre disposition particulièrement avantageuse de ce mode de réalisation, le baculovirus modifié contient la séquence codant pour la β-galactosidase, placée sous le contrôle du promoteur actif (protéine P10 ou polyédrine). Les baculovirus modifiés de la sorte ont la propriété de former des plages virales bleues en présence du substrat X-gal.

L'utilisation de baculovirus obtenus conformément à l'une des deux dispositions qui précèdent permet, lors de l'insertion ultérieure d'un gène étranger, de sélectionner aisément les virus recombinants ayant intégré ledit gène étranger à l'intérieur du gène de la polyédrine ou de celui de la β-galactosidase. En effet, dans le premier cas, lesdits virus recombinants ne forment plus de polyèdres ; dans le deuxième cas, ils forment des plages blanches, au lieu des plages bleues.

Pour obtenir des baculovirus modifiés utilisables pour l'obtention des vecteurs d'expression conformes à l'Invention, l'on procède à l'inactivation, par tous moyens appropriés, de l'un des promoteurs tardifs forts présents dans le génome du baculovirus sauvage.

L'on procède par exemple de la sorte à l'inactivation du promoteur de la polyédrine, ou du promoteur de la protéine P10 par excision d'un fragment d'ADN comprenant ledit promoteur.

L'excision dudit fragment d'ADN peut être effectuée à l'aide d'enzymes de restriction.

Il va de soi que d'autres procédés, comme par exemple, des procédés classiques faisant appel à des techniques de recombinaison homologue, peuvent être utilisés pour procéder à l'excision du fragment d'ADN désiré.

L'inactivation de l'un ou l'autre des deux promoteurs peut également être effectuée par mutagénèse de séquences participant à l'activité dudit promoteur, par exemple comme décrit dans la publication de RAKIN et al. précitée.

Des vecteurs d'expression chargés conformes à l'Invention, peuvent être obtenus à partir des baculovirus modifiés, par divers procédés connus en eux-mêmes, par exemple, et de façon non limitative, en utilisant des vecteurs de transfert, tels que ceux décrits dans les Demandes de Brevet Européen 127 839 et 340 359, ou bien par insertion directe, comme décrit dans la Demande de Brevet Européen 345 152).

La séquence codant pour la protéine exogène que l'on désire faire exprimer est insérée sous contrôle du promoteur actif ; la séquence qui, chez le baculovirus sauvage est normalement placée sous contrôle dudit promoteur, peut être totalement ou partiellement excisée, et remplacée par la séquence codant pour la protéine exogène.

La présente invention sera mieux comprise à l'aide du complément de description qui va suivre, qui se réfère à des exemples de construction des baculovirus modifiés et des vecteurs d'expression conformes à l'invention. Il va toutefois de soi que ces exemples sont donnés uniquement à titre d'illustration de la présente invention dont ils ne constituent en aucune manière une limitation.

### EXEMPLE 1 : Construction d'un baculovirus modifié dépourvu du promoteur et du gène de structure de la polyédrine

Les protocoles utilisées dans cet exemple et les suivants font appel à des techniques classiques du génie génétique, telles que celles décrites par MANIATIS et al.[Molecular cloning : A Laboratory Manual ; Cold Spring Harbor Laboratory, 1982]. Les conditions particulières à chaque expérimentation sont, s'il y a lieu, précisées dans l'exemple correspondant.

Des baculovirus modifiés conformes à l'Invention sont obtenus par digestion partielle de l'ADN du baculovirus sauvage par deux enzymes de restriction, dont les sites de coupure se situent pour l'une d'entre elles, en amont du promoteur de la polyédrine, et pour l'autre, en aval dudit promoteur, soit dans la séquence codant pour la polyédrine, soit en aval de ladite séquence.

L'ADN génomique total d'un baculovirus appartenant à la souche *Autographa californica* est coupé successivement par les enzymes EcoRV et Eco47III suivant la technique de digestion partielle décrite dans "Current Protocols in Molecular Biology"; Ausubel et al. Eds. ; publié par Geene Publishing Associates and Wiley Interscience (paragraphe 3.1.6.).

Les conditions choisies sont les suivantes :

### Coupure par EcoRV

10 µg d'ADN de Baculovirus sont dilués dans 100 µl de tampon Tris HCl 10 mM (pH 7,5), 10 mM MgCl2, 150 mM NaCl

3 unités d'enzyme par µg d'ADN sont ajoutées. Des dilutions successives sont réalisées dans le même tampon, et pour chaque dilution, après une incubation de 15 minutes à 37 °C, les produits de restriction sont analysés sur gel d'agarose afin de déterminer la dilution qui ne donne qu'une coupure par molécule d'ADN viral.

La dilution retenue est, pour Eco RV, de 1/27.

### Coupure par Eco 47 III

Les conditions expérimentales sont les mêmes que celles décrites pour Eco RV.

La dilution retenue est de 1/54.

Les molécules coupées par EcoRV et Eco47III sont pourvues d'extrémités franches, ce qui permet de procéder directement à leur ligation pour obtenir des molécules d'ADN viral circulaire..

Les molécules d'ADN viral résultantes sont utilisées, après purification, pour transfecter des cultures de cellules de *Spodoptera frugiperda* (Souche SF9, ATCC n° CRL 1711), selon le protocole décrit par GRAHAM et VAN DER ERB, [Virology, 52, 305 - 309 (1973)].

Dans ces conditions, les molécules d'ADN portant la délétion souhaitée sont infectieuses, et donnent des virus qui produisent des plages ne formant pas de polyèdres.

Ces virus sont appelés virus pob⁻.

### EXEMPLE 2

L'ADN génomique du baculovirus *Autographa Californica* est digéré successivement par les enzymes KpnI et EcoRV, dans les conditions décrites à l'exemple I, à ceci près que le traitement par Kpn I est effectué dans un tampon sans NaCl, et à une dilution de 1/9. Les molécules sont ensuite religuées et utilisées pour transfecter des cultures de *Spodoptera Frugiperda,* comme décrit précédemment.

### EXEMPLE 3

On procède comme décrit dans les exemples 1 ou 2, à ceci près que le baculovirus sauvage utilisé appartient à la souche *Galleria mellonella*.

### EXEMPLE 4 : Insertion de la séquence codant pour la β-galactosidase sous contrôle du promoteur de la protéine P10

Le fragment de restriction Eco R1-P, obtenu à partir du génome du baculovirus *Autographa Californica* sauvage, et qui contient le gène de structure de la protéine P10, est inséré dans le lieur multisite du plasmide pUC9 au site Eco R1. Ce fragment contient un site unique BglII, situé à +152 pb par rapport au codon ATG du gène P10. Un fragment BamHI comprenant la majeure partie du gène de la β-galactosidase, (qui possède un site BamHI en aval de son propre ATG) est inséré au site BglII ; les cadres de lecture du début de la protéine P10 et de la β-galactosidase sont ainsi en phase.

Le plasmide obtenu (D3PZ) possède donc le début du gène de la protéine P10, et le gène de la β-galactosidase. On cotransfecte des cellules de *Spodoptera frugiperda* avec le plasmide D3PZ et l'ADN du virus pob⁻ (1 µg de D3PZ ; 0,5 µg d'ADN de pob- ; le protocole est identique à celui de l'exemple 1) et on sélectionne les baculovirus recombinants (pob⁻ β-galactosidase), qui forment des plages bleues en présence de X gal. Les virus recombinants obtenus expriment la β-galactosidase en quantité très importante, comme le montre l'exemple 7 ci-dessous.

### EXEMPLE 5 : Introduction du gène de structure de la polyédrine à la place du gène de structure de la protéine P10

Le fragment Eco R1-I de l'ADN génomique du baculovirus d'*Autographa californica,* contenant le gène de la polyédrine a été cloné dans le lieur multisite du plasmide pUC18 au site EcoRI. Un site PvuII a été introduit, à -2 bases par rapport au codon ATG de la séquence codant pour la polyédrine.

D'autre part, un plasmide de contenant le fragment Eco R1-P du génome du baculovirus d'*Autographa californica* (cf. exemple 4) est ouvert au site BglII. Après action de l'enzyme Bal31, puis de la polymérase de Klénow un linker BglII est introduit. On obtient ainsi un plasmide appelé pGH 80-82, dépourvu de la majeure partie de la séquence codant pour la protéine P10, et portant un site BglII en position -4 par rapport à l'ATG de la protéine P10.

Le gène de structure de la polyédrine, excisé du plasmide pUC18 à l'aide des enzymes de restriction PvuII et Eco47III, est introduit au site BglII du plasmide pGH 80-82, après restauration des extrémités franches par la polymérase de Klénow.

Le plasmide obtenu, appelé pGH 80-82 ob⁺ est utilisé, avec le virus pob⁻ obtenu à l'Exemple 1, pour cotransfecter des cellules de *Spodoptera frugiperda.*

Les virus recombinants (pob⁻ ob⁺), qui forment des polyèdres, sont sélectionnés.

Une souche de virus recombinants (pob⁻ob⁺) a été déposée le 17 Juillet 1990, auprès de la Collection Nationale de Microorganismes, sous le numéro I-978.

### EXEMPLE 6 : Clonage du gène de l'Acétylcholinestérase (Ache) de Drosophile

Un fragment d'ADNc obtenu à partir de l'ARNm de l'Ache a été cloné au préalable dans un plasmide pEMBL8 entre les sites Sma I et Eco R1. A partir dudit plasmide, le gène de l'Ache est excisé par l'action des enzymes FspI (site à -14 pb du codon ATG de l'Ache) et SacI (site à + 117 pb du codon de terminaison de l'Ache).

Après traitement par l'ADN polymérase du phage T₄, le fragment obtenu est inséré dans le plasmide pGH 80-22, au site Bgl II, dont les extrémités ont été au préalable rendues franches par la polymérase de Klenow. Le plasmide obtenu est appelé pGH 80-22 Ache.

Ce plasmide est utilisé
- ou bien pour cotransfecter des cellules avec l'ADN d'un virus pob⁻ ob⁺ ; on sélectionne alors les recombinants qui ne forment plus de polyèdres
- ou bien pour cotransfecter des cellules avec l'ADN d'un virus pob⁻ β-galactosidase : dans ce cas, les virus recombinants sélectionnés seront ceux qui forment des plages blanches.

### EXEMPLE 7 : Expression du gène de la β-galactosidase : Comparaison entre des baculovirus recombinants connus dans l'art antérieur et les vecteurs d'expression conformes à l'Invention.

Trois constructions ont été réalisées :
- le gène de la β-galactosidase est "fusionné" à celui de la polyédrine (Polyédrine-β-Gal.). Le virus recombinant est de type classique (il possède les deux promoteurs forts) ;
- le gène de la β-galactosidase est fusionné à celui du polypeptide P10 (Acp10Z). Le virus recombinant, est de type classique (il possède les deux promoteurs forts et fabrique des polyèdres) ;
- le gène de la β-galactosidase a été fusionné à celui du polypeptide P10 (D3PZ). Le virus recombinant, de type pob⁻, ne possède plus le promoteur ni le gène de la polyédrine.
Ces différents virus recombinants ont été utilisés pour transfecter des cellules de *Spodoptera frugiperda.*
Aux temps suivants : 24h, 48h, 72h après infection l'activité β-galactosidase est mesurée dans le surnageant de culture et dans les cellules elles-mêmes en utilisant l'ONPG comme substrat chromogène. 3 mesures sont faites sur chaque point.

Les résultats sont résumés dans le Tableau I suivant :

**TABLEAU I.**

| | 24 h | | 48 H | | 72 h | |
|---|---|---|---|---|---|---|
| | Sn | Cel | Sn | Cel | Sn | Cel |
| Polyédrine β-Gal | 0 | 225 | 770 | 17133 | 2735 | 36732 |
| | | ±15 | ±25 | ±1400 | ±600 | ±1133 |
| Ac P10Z | 0 | 275 | 235 | 19066 | 1105 | 42999 |
| | | ±10 | ±20 | ±600 | ±210 | ±4135 |
| D3PZ | 0 | 300 | 295 | 24733 | 2080 | 46532 |
| | | ±15 | ±70 | ±640 | ±1200 | ±2400 |
| (Unité β-Gal = 1000 (DO₄₂₀)/temps (min) x volume (ml) Cel = Cellules Sn = Surnageant | | | | | | |

48 heures après transfection, le surnageant de culture renferme très peu d'activité (il n'y a pas encore de lyse cellulaire). Les cellules D3PZ renferment 1,4 fois plus d'activité que les cellules Polyédrine β-Gal. A 72 heures malgré une lyse cellulaire qui devient importante, D3PZ possède toujours plus d'activité que les autres constructions.

### EXEMPLE 8 - Construction d'un baculovirus modifié dépourvu du promoteur et du gène de structure de la protéine P10

L'ADN génomique total d'un baculovirus appartenant à la souche *Autographa californica* est coupé successivement par les enzymes XhoI et BglII, suivant la technique de digestion partielle indiquée à l'exemple 1. Les conditions mises en oeuvre sont les suivantes :

### Coupure par XhoI

10 µg d'ADN de Baculovirus sont dilués dans 100 µl de tampon Tris HCl 50 mM (pH 7,5), 10 mM MgCl₂, 100 mM NaCl.

3 unités d'enzyme par µg d'ADN sont ajoutées. Des dilutions successives sont réalisées dans le même tampon, et pour chaque dilution, après une incubation de 15 minutes à 37 °C, les produits de restriction sont analysés sur gel d'agarose afin de déterminer la dilution qui ne donne qu'une coupure par molécule d'ADN viral.

La dilution retenue est, pour XhoI, de 1/27.

### Coupure par BglII

10 µg d'ADN de Baculovirus sont dilués dans 100 µl de tampon Tris HCl 10 mM (pH 7,5), 10 mM MgCl, 50 mM NaCl.

Les autres conditions expérimentales sont les mêmes que celles décrites pour XhoI.

La dilution retenue est de 1/54.

Les extrémités des molécules coupées par XhoI et BglII sont réparées par l'enzyme de Klenow avant de procéder à leur ligation.

Les molécules d'ADN viral résultantes sont utilisées pour transfecter des cultures de cellules de *Spodoptera frugiperda.*

Dans ces conditions, les molécules d'ADN portant la délétion souhaitée sont infectieuses.

Ces virus sont appelés virus P1O⁻ ; ils produisent plus de polyédrine que le virus sauvage^{.}

### EXEMPLE 9 - Insertion du gène de la β-galactosidase dans un baculovirus modifié conforme à l'Invention

Un vecteur de transfert obtenu à partir d'un plasmide pUC8, et comprenant le fragment EcoR1-I du baculovirus (Cf. Exemple 5), qui contient un site unique Bam HI, à +171 bases du codon ATG du gène de la polyédrine, est utilisé pour cette construction.

Un fragment Bam HI du gène de la β-galactosidase (Cf. Exemple 4) est inséré au site Bam HI dudit vecteur de transfert.

Les vecteurs ainsi obtenus sont utilisés, en même temps que des baculovirus modifiés P10⁻, obtenus par le protocole décrit dans l'exemple 8, pour cotransfecter les cellules de *Spodoptera frugiperda.*

Les baculovirus ayant intégré le gène de la β-galactosidase ne forment plus de polyèdres, mais synthétisent la β-galactosidase, facilement détectable et quantifiable par coloration spécifique avec le substrat chromogène X-gal.

La production de β-galactosidase par les baculovirus modifiés ainsi obtenus a été comparée, comme décrit à l'exemple 7, avec celle d'une construction similaire obtenue à partir de baculovirus "sauvages" (possédant le promoteur de la protéine P10 et celui de la polyédrine). Le taux de β-galactosidase produit 48 heures après infection, est de 25% plus élevé chez les baculovirus modifiés dépourvus du promoteur de la protéine P10 que chez les baculovirus sauvages.

## Revendications (Revendications pour l'(les) Etat(s) contractant(s) suivant(s): AT, BE, CH, DE, DK, FR, GB, IT, LI, LU, NL, SE)

1. Vecteurs d'expression, caractérisés en ce qu'ils sont obtenus à partir d'un baculovirus modifié dans lequel un des deux promoteurs tardifs forts présents dans le génome du baculovirus sauvage est inactif, et en ce que la séquence placée sous le contrôle du promoteur tardif fort actif dans ledit baculovirus modifié est différente de la séquence correspondante du baculovirus sauvage.

2. Vecteur d'expression selon la Revendication 1, caractérisé en ce que le promoteur inactif est celui du gène de la polyédrine et le promoteur actif est celui du gène de la protéine P10.

3. Vecteur d'expression selon la Revendication 1, caractérisé en ce que le promoteur inactif est celui du gène de la protéine P10, et le promoteur actif est celui du gène de la polyédrine.

4. Baculovirus modifié, utilisable pour l'obtention d'un vecteur d'expression, selon la revendication 2, caractérisé en ce que son génome est dépourvu d'une séquence d'ADN comprise entre le site EcoRV situé à -95 pb et le site SspI situé à +910 pb du codon d'initiation ATG de la séquence de la polyédrine.

5. Baculovirus modifié, utilisable pour l'obtention d'un vecteur d'expression, selon la revendication 2, caractérisé en ce que son génome est dépourvu d'une séquence d'ADN comprise entre le site EcoRV situé à -95 pb et le site KpnI situé à +633 pb du codon d'initiation ATG de la séquence de la polyédrine.

6. Baculovirus modifié, utilisable pour l'obtention d'un vecteur d'expression, selon la revendication 2, caractérisé en ce que son génome est dépourvu d'une séquence d'ADN comprise entre le site EcoRV situé à -95 pb et le site Eco47III situé à +733 pb du codon d'initiation ATG de la séquence codant pour la polyédrine.

7. Baculovirus modifié utilisable pour l'obtention de vecteurs d'expression selon la Revendication 3, caractérisé en ce que son génome est dépourvu du promoteur du gène de la protéine P10.

8. Baculovirus modifié selon la revendication 7, caractérisé en que son génome est dépourvu d'une séquence d'ADN comprise entre le site XhoI situé à +569pb du codon d'initiation ATG de la séquence codant pour le polypeptide P26 et le site BglII, situé à +152pb du codon d'initiation ATG de la séquence codant pour le polypeptide P10.

9. Vecteur d'expression, selon l'une quelconque des revendications 1 à 3, caractérisé en ce qu'il contient une séquence marqueur, placée sous le contrôle du promoteur actif.

10. Vecteur d'expression, selon la revendication 9, caractérisé en ce que la séquence marqueur est la séquence codant pour la polyédrine, laquelle séquence est placée sous le contrôle du promoteur du gène de la protéine P10.

11. Souche de baculovirus modifié, constituant un vecteur d'expression, selon la Revendication 10, laquelle souche a été déposée en date du 17 Juillet 1990 auprès de la Collection Nationale de Microorganismes, sous le numéro de dépôt I-978.

12. Vecteur d'expression, selon la revendication 9, caractérisé en ce que la séquence marqueur est la séquence codant pour la β-galactosidase.

## Revendications (Revendications pour l'(les) Etat(s) contractant(s) suivant(s): ES, GR)

1. Procédé d'obtention de vecteurs d'expression, à partir d'un baculovirus modifié dans lequel un des deux promoteurs tardifs forts présents dans le génome du baculovirus sauvage est inactif, caractérisé en ce que l'on place sous le contrôle du promoteur tardif fort actif dans ledit baculovirus modifié une séquence différente de la séquence correspondante du baculovirus sauvage.

2. Procédé selon la Revendication 1, caractérisé en ce que le promoteur inactif est celui du gène de la polyédrine et le promoteur actif est celui du gène de la protéine P10.

3. Procédé selon la Revendication 1, caractérisé en ce que le promoteur inactif est celui du gène de la protéine P10, et le promoteur actif est celui du gène de la polyédrine.

4. Procédé d'obtention d'un baculovirus modifié utilisable pour la mise en oeuvre du procédé selon la revendication 2, caractérisé en ce que l'on effectue la délétion d'une séquence d'ADN comprise entre le site EcoRV situé à -95 pb et le site SspI situé à +910 pb du codon d'initiation ATG de la séquence de la polyédrine.

5. Procédé d'obtention d'un baculovirus modifié utilisable pour la mise en oeuvre du procédé selon la revendication 2, caractérisé en ce que l'on effectue la délétion d'une séquence d'ADN comprise entre le site EcoRV situé à -95 pb et le site KpnI situé à +633 pb du codon d'initiation ATG de la séquence de la polyédrine.

6. Procédé d'obtention d'un baculovirus modifié utilisable pour la mise en oeuvre du procédé selon la revendication 2, caractérisé en ce que l'on effectue la délétion d'une séquence d'ADN comprise entre le site EcoRV situé à -95 pb et le site Eco47III situé à +733 pb du codon d'initiation ATG de la séquence codant pour la polyédrine.

7. Procédé d'obtention d'un baculovirus modifié utilisable pour la mise en oeuvre du procédé selon la revendication 3, caractérisé en ce que l'on effectue la délétion du promoteur du gène de la protéine P10.

8. Procédé selon la revendication 7, caractérisé en ce que l'on effectue la délétion d'une séquence d'ADN comprise entre le site XhoI situé à +569pb du codon d'initiation ATG de la séquence codant pour le polypeptide P26 et le site BglII, situé à +152pb du codon d'initiation ATG de la séquence codant pour le polypeptide P10.

9. Procédé selon une quelconque des revendications 1 à 3, caractérisé en ce que l'on place une séquence marqueur sous le contrôle du promoteur actif.

10. Procédé selon la revendication 9, caractérisé en ce que ladite séquence marqueur est la séquence codant pour la polyédrine, laquelle séquence est placée sous le contrôle du promoteur du gène de la protéine P10.

11. Procédé selon la revendication 10, caractérisé en ce que le vecteur d'expression obtenu est constitué par la souche de baculovirus modifié qui été déposée en date du 17 Juillet 1990 auprès de la Collection Nationale de Microorganismes, sous le numéro de dépôt I-978.

12. Procédé selon la revendication 9, caractérisé en ce que ladite séquence marqueur est la séquence codant pour la β-galactosidase.

## Patentansprüche (Patentansprüche für folgende(n) Vertragsstaat(en): AT, BE, CH, LI, DE, DK, FR, GB, IT, LU, NL, SE)

1. Expressionsvektoren, dadurch **gekennzeichnet,** daß sie aus einem modifizierten Baculovirus erhalten wurden, in dem einer der in dem Genom des Wildtyp Baculovirus vorhandenen zwei späten starken Promotoren inaktiv ist, und daß die Sequenz, die unter der Kontrolle des aktiven späten starken Promotors in dem modifizierten Baculovirus steht, sich von der entsprechenden Sequenz des Wildtyp-Baculovirus unterscheidet.

2. Expressionsvektor nach Anspruch 1, dadurch **gekennzeichnet,** daß der inaktive Promotor der des Polyhedringens ist, und der aktive Promotor der des Gens des Proteins P10 ist.

3. Expressionsvektor nach Anspruch 1, dadurch **gekennzeichnet,** daß der inaktive Promotor der des Gens des Proteins P10 ist, und der aktive Promotor der des Polyhedringens ist.

4. Modifiziertes Baculovirus, zur Verwendung zum Erhalt eines Expressionsvektors nach Anspruch 2, dadurch **gekennzeichnet,** daß seinem Genom eine DNA-Sequenz, umfassend den Abschnitt zwischen der EcoRV-Stelle, die bei -95 bp und der Sspl-Stelle, die bei +910 bp des Initiationscodons ATG der Polyhedrinsequenz gelegen ist, fehlt.

5. Modifiziertes Baculovirus zur Verwendung zum Erhalt eines Expressionsvektors nach Anspruch 2, dadurch **gekennzeichnet,** daß seinem Genom eine DNA-Sequenz, umfassend den Abschnitt zwischen der EcoRV-Stelle, die bei -95 bp und der KpnI-Stelle, die bei +633 bp des Initiationscodons ATG der Polyhedrinsequenz gelegen ist, fehlt.

6. Modifiziertes Baculovirus zur Verwendung zum Erhalt eines Expressionsvektors nach Anspruch 2, dadurch **gekennzeichnet,** daß seinem Genom eine DNA-Sequenz, umfassend den Abschnitt zwischen der EcoRV-Stelle, die bei -95 bp und der Eco47III-Stelle, die bei +733 bp des Initiationscodons ATG der Polyhedrin-codierenden Sequenz gelegen ist, fehlt.

7. Modifiziertes Baculovirus zur Verwendung zum Erhalt von Expressionsvektoren nach Anspruch 3, dadurch **gekennzeichnet,** daß seinem Genom der Promotor des Gens des Proteins P10 fehlt.

8. Modifiziertes Baculovirus nach Anspruch 7, dadurch **gekennzeichnet,** daß seinem Genom eine DNA-Sequenz, umfassend den Abschnitt zwischen der XhoI-Stelle, die bei +569 bp des Initiationscodons ATG, der das Polypeptid P26 codierenden Sequenz gelegen ist, und der BglII-Stelle, die bei +152 bp des Initiationscodons ATG der das Polypeptid P10 codierenden Sequenz gelegen ist, fehlt.

9. Expressionsvektor nach einem der Ansprüche 1 bis 3, dadurch **gekennzeichnet,** daß er eine Markersequenz, die unter der Kontrolle des aktiven Promotors steht, enthält.

10. Expressionsvektor nach Anspruch 9, dadurch **gekennzeichnet,** daß die Markersequenz die Polyhedrin-codierende Sequenz ist, wobei die Sequenz unter der Kontrolle des Promotors des Gens des Proteins P10 steht.

11. Modifizierter Baculovirusstamm als Expressionsvektor nach Anspruch 10, wobei der Stamm am 17. Juli 1990 bei der Collection Nationale de Microorganismes unter der Hinterlegungsnummer I-978 hinterlegt wurde.

12. Expressionsvektor nach Anspruch 9, dadurch **gekennzeichnet,** daß die Markersequenz die β-Galaktosidase-codierende Sequenz ist.

## Patentansprüche (Patentansprüche für folgende(n) Vertragsstaat(en): ES, GR)

1. Verfahren zur Produktion von Expressionsvektoren aus einem modifizierten Baculovirus, in dem einer der in dem Genom des Wildtyp-Baculovirus vorhandenen zwei starken späten Promotoren inaktiv ist, dadurch **gekennzeichnet,** daß man eine Sequenz, die sich von der entsprechenden Sequenz des Wildtyp-Baculovirus unterscheidet, unter die Kontrolle des aktiven starken späten Promotors in dem modifizierten Baculovirus einbringt.

2. Verfahren nach Anspruch 1, dadurch **gekennzeichnet,** daß derinaktive Promotor der des Polyhedringens ist, und daß der aktive Promotor der des Gens des Proteins P10 ist.

3. Verfahren nach Anspruch 1, dadurch **gekennzeichnet,** daß der inaktive Promotor der des Gens des Proteins P10 ist, und der aktive Promotor der des Polyhedringens ist.

4. Verfahren zur Produktion eines modifizierten Baculovirus zur Verwendung zur Durchführung des Verfahrens nach Anspruch 2, dadurch **gekennzeichnet,** daß man eine Deletion einer DNA-Sequenz, umfassend den Abschnitt zwischen der EcoRV-Stelle, die bei -95 bp und der SspI-Stelle, die bei +910 bp des Initiationscodons ATG der Polyhedrinsequenz gelegen ist, durchführt.

5. Verfahren zur Produktion eines modifizierten Baculovirus zur Verwendung zur Durchführung des Verfahrens nach Anspruch 2, dadurch **gekennzeichnet,** daß man eine Deletion einer DNA-Sequenz, umfassend den Abschnitt zwischen der EcoRV-Stelle, die bei -95 bp und der KpnI-Stelle, die bei +633 bp des Initiationscodons ATG der Polyhedrinsequenz gelegen ist, durchführt.

6. Verfahren zur Produktion eines modifizierten Baculovirus zur Verwendung zur Durchführung des Verfahrens nach Anspruch 2, dadurch **gekennzeichnet,** daß man eine Deletion einer DNA-Sequenz, umfassend den Abschnitt zwischen der EcoRV-Stelle, die bei -95 bp und der Eco47III-Stelle, die bei +733 bp des Initiationscodons ATG der Polyhedrin-codierenden Sequenz gelegen ist, durchführt.

7. Verfahren zur Produktion eines modifizierten Baculovirus zur Verwendung zur Durchführung des Verfahrens nach Anspruch 3, dadurch **gekennzeichnet,** daß man eine Deletion des Promotors des Gens des Proteins P10 durchführt.

8. Verfahren nach Anspruch 7, dadurch **gekennzeichnet,** daß man die Deletion einer DNA-Sequenz, umfassend den Abschnitt zwischen der Xhol-Stelle, die bei +569 bp des Initiationscodons ATG der das Polypeptid P26 codierenden Sequenz gelegen ist und der BglII-Stelle, die bei +152 bp des Initiationscodons ATG der das Polypeptid P10 codierenden Sequenz gelegen ist, durchführt.

9. Verfahren nach einem der Ansprüche 1 bis 3, dadurch **gekennzeichnet,** daß man eine Markersequenz unter die Kontrolle des aktiven Promotors einbringt.

10. Verfahren nach Anspruch 9, dadurch **gekennzeichnet,** daß die Markersequenz die Sequenz ist, die Polyhedrin codiert, wobei die Sequenz unter die Kontrolle des Promotors des Gens des Proteins P10 gebracht wurde.

11. Verfahren nach Anspruch 10, dadurch **gekennzeichnet,** daß der so erhaltene Expressionsvektor aus dem modifizierten Baculovirusstamm besteht, der am 17. Juli 1990 bei der Collection Nationale de Microorganismes unter der Hinterlegungsnummer 1-978 hinterlegt wurde.

12. Verfahren nach Anspruch 9, dadurch **gekennzeichnet,** daß die Markersequenz die Sequenz ist, die β-Galaktosidase codiert.

## Claims (Claims for the following Contracting State(s): AT, BE, CH, LI, DE, DK, FR, GB, IT, LU, NL, SE)

1. Expression vectors, characterised in that they are obtained from a modified baculovirus in which one of the two strong late promoters present in the genome of the wild-type baculovirus is inactive, and in that the sequence placed under the control of the strong late promoter which is active in the said modified baculovirus is different from the corresponding sequence of the wild-type baculovirus.

2. Expression vector according to Claim 1, characterised in that the inactive promoter is that of the polyhedrin gene and the active promoter is that of the P10 protein gene.

3. Expression vector according to Claim 1, characterised in that the inactive promoter is that of the P10 protein gene and the active promoter is that of the polyhedrin gene.

4. Modified baculovirus, usable for obtaining an expression vector according to Claim 2, characterised in that its genome is devoid of a DNA sequence bounded by the EcoRV site located at -95 bp and the SspI site located at +910 bp from the ATG initiation codon of the polyhedrin sequence.

5. Modified baculovirus, usable for obtaining an expression vector according to Claim 2, characterised in that its genome is devoid of a DNA sequence bounded by the EcoRV site located at -95 bp and the KpnI site located at +633 bp from the ATG initiation codon of the polyhedrin sequence.

6. Modified baculovirus, usable for obtaining an expression vector according to Claim 2, characterised in that its genome is devoid of a DNA sequence bounded by the EcoRV site located at -95 bp and the Eco47III site located at +733 bp from the ATG initiation codon of the sequence coding for polyhedrin.

7. Modified baculovirus, usable for obtaining expression vectors according to Claim 3, characterised in that its genome is devoid of the P10 protein gene promoter.

8. Modified baculovirus according to Claim 7, characterised in that its genome is devoid of a DNA sequence bounded by the XhoI site located at +569 bp from the ATG initiation codon of the sequence coding for the P26 polypeptide and the BglII site located at +152 bp from the ATG initiation codon of the sequence coding for the P10 polypeptide.

9. Expression vector according to any one of Claims 1 to 3, characterised in that it contains a marker sequence, placed under the control of the active promoter.

10. Expression vector according to Claim 9, characterised in that the marker sequence is the sequence coding for polyhedrin, which sequence is placed under the control of the P10 protein gene promoter.

11. Modified baculovirus strain, constituting an expression vector according to Claim 10, which strain was deposited on 17th July 1990 with the Collection Nationale de Microorganismes (National Collection of Microorganisms) under deposit number I-978.

12. Expression vector according to Claim 9, characterised in that the marker sequence is the sequence coding for β-galactosidase.

## Claims (Claims for the following Contracting State(s): ES, GR)

1. Method for obtaining expression vectors from a modified baculovirus in which one of the two strong late promoters present in the genome of the wild-type baculo-virus is inactive, characterised in that a sequence which is different from the corresponding sequence of the wild-type baculovirus is placed under the control of the strong late promoter which is active in the said modified baculovirus.

2. Method according to Claim 1, characterised in that the inactive promoter is that of the polyhedrin gene and the active promoter is that of the P10 protein gene.

3. Method according to Claim 1, characterised in that the inactive promoter is that of the P10 protein gene and the active promoter is that of the polyhedrin gene.

4. Method for obtaining a modified baculovirus usable for implementing the method according to Claim 2, characterised in that a DNA sequence bounded by the EcoRV site located at -95 bp and the SspI site located at +910 bp from the ATG initiation codon of the polyhedrin sequence is deleted.

5. Method for obtaining a modified baculovirus usable for implementing the method according to Claim 2, characterised in that a DNA sequence bounded by the EcoRV site located at -95 bp and the KpnI site located at +633 bp from the ATG initiation codon of the polyhedrin sequence is deleted.

6. Method for obtaining a modified baculovirus usable for implementing the method according to Claim 2, characterised in that a DNA sequence bounded by the EcoRV site located at -95 bp and the Eco47III site located at +733 bp from the ATG initiation codon of the sequence coding for polyhedrin is deleted.

7. Method for obtaining a modified baculovirus usable for implementing the method according to Claim 3, characterised in that the P10 protein gene promoter is deleted.

8. Method according to Claim 7, characterised in that a DNA sequence bounded by the XhoI site located at +569 bp from the ATG initiation codon of the sequence coding for the P26 polypeptide and the BglII site located at +152 bp from the ATG initiation codon of the sequence coding for the P10 polypeptide is deleted.

9. Method according to any one of Claims 1 to 3, characterised in that a marker sequence is placed under the control of the active promoter.

10. Method according to Claim 9, characterised in that the said marker sequence is the sequence coding for polyhedrin, which sequence is placed under the control of the P10 protein gene promoter.

11. Method according to Claim 10, characterised in that the expression vector obtained consists of the modified baculovirus strain which was deposited on 17th July 1990 with the Collection Nationale de Microorganismes (National Collection of Microorganisms) under deposit number I-978.

12. Method according to Claim 9, characterised in that the said marker sequence is the sequence coding for β-galactosidase.
